(19) Europäisches Patentamt
European Patent Office
Office européen des brevets

(11) Publication number: **0 456 988 B1**

# EUROPEAN PATENT SPECIFICATION

(45) Date of publication of patent specification: **11.10.95**   (51) Int. Cl.6: **A61K 31/19**

(21) Application number: **91104114.3**

(22) Date of filing: **16.03.91**

(54) **Use of naproxen as mydriatic agent.**

(30) Priority: **18.05.90 IT 2036690**

(43) Date of publication of application:
**21.11.91 Bulletin 91/47**

(45) Publication of the grant of the patent:
**11.10.95 Bulletin 95/41**

(84) Designated Contracting States:
**AT BE CH DE DK ES FR GB GR IT LI LU NL SE**

(56) References cited:

**JOURNAL OF OCULAR PHARMACOLOGY, vol. 7, no. 2, 1991, pages 125-134, Mary Ann-Liebert, Inc.; S. SPAMPINATO et al.: "Effects of sodium naproxen eye drops onrabbit ocular inflammation induced by sodium arachidonate"**

**IDEM**

**K. SASAKI et al.: "Developments in Ophthalmology", vol. 21. DISTRIBUTION OFCATARACTS IN THE POPULATION AND INFLUENCING FACTORS;SATELLITE MEETING ON CATARACT AND EPIDEMIOLOGY HELD IN**

(73) Proprietor: **S.I.F.I. Società Industria Farmaceutica Italiana S.p.A.
Via Nicola Coviello 15/B
I-95128 Catania (IT)**

(72) Inventor: **Benanti, Giuseppe
Via Barriera del Bosco 9/a
I-95030 Sant'Agata Li Battiati,
Catania (IT)**
Inventor: **Bucolo, Claudio
Via Cesare Beccaria 100
I-95123 Catania (IT)**

(74) Representative: **Giambrocono, Alfonso, Dr. Ing. et al
Ing. A. Giambrocono & C. S.r.l.
Via Rosolino Pilo 19/B
I-20129 Milano (IT)**

AFFILIATION WITH THE XXVI INTERNATION-
AL CONGRESS OFOPHTHALMOLOGY, Singa-
pore, Singapore, March 1990, Karger
AG,Basel, CH, 1991, pages 170-178; S.K.
GUPTA et al.: "Naproxen:an aldose reduc-
tase inhibitor and potential anti-catarac-
tagent"

IDEM

TRANS. AMER. OPHTHAL. SOC., vol. LXXIV,
no. 74, 1977, pages 637-660, US; S.M.PODOS:
"Prostaglandins, nonsteroidal anti-inflamma-
tory agents and eye disease"

IDEM

K. SASAKI et al.: "Developments in Ophthal-
mology", vol. 21. DISTRIBUTION OF-
CATARACTS IN THE POPULATION AND IN-
FLUENCING FACTORS;SATELLITE MEETING
ON CATARACT AND EPIDEMIOLOGY HELD
INAFFILIATION WITH THE XXVI INTERNA-
TIONAL CONGRESS OFOPHTHALMOLOGY, Si
ngapore, Singapore, March 1990, Karger
AG,Basel, CH, 1991, pages 151-156; S.K.
GUPTA et al.:"Relationship between aldose
reductase inhibiting activity

and anti-cataract action of various non-
steroidal anti-inflammatory drugs"

IDEM

PATENT ABSTRACTS OF JAPAN, vol. 8, no.
118 (C-226), 31st May 1984;& JP-A-59 029 616
(KAKEN SEIYAKU K.K.) 16-02-1984

PATENT ABSTRACTS OF JAPAN, vol. 7, no.
235 (C-191), 19th October 1983;& JP-A-58 126
810 (KAKEN KAGAKU K.K.) 28-07-1983

OPHTHALMOLOGY, vol. 86, no. 1, January
1979, pages 126-130; F.T. FRAUNFEL-
DER:"Interim report: national registry of pos-
sible drug-induced ocular sideeffects"

ACTA OPHTHALMOL., vol. 63, no. 5, October
1985, pages 519-524, DK; P.M.PRITCHETT et
al.: "The effect of naproxen on the ocular
inflammatory responsefollowing ex-
tracapsular lens extraction in rabbits"

**Description**

The present invention relates to the use of naproxen sodium salt for the manufacture of a medicine useful in the maintainment of eye mydriasis.

Naproxen is a non-steroidal analgesic, antipyretic and antiinflammatory agent belonging to the acid phenylacetic derivatives class and described in US-A-3978116 and US-A-3980699 (Syntex).

The use of naproxen and salts thereof in the ophthalmic field has been already described in various patents.

DE-A-3026402 (Syntex) discloses the use of naproxen in treating diabetic retinopathies by inhibiting the new blood capillaries and vessels formation. BE 885441 (Kahani), EP 244315 (Lab. Chauvin-Blache), BE 903568 (Pulitzer Italiana) and EP 105635 (Alcon Lab. Inc.) disclose naproxen formulations being able, by employing particular naproxen salts or specific adjuvants, to make naproxen to be dissolved, thereby it may be used also in the ophthalmic field, but solely as antiinflammatory agent.

It is known that during certain ophthalmic surgical operations, e.g. cataractous lens extra- or intra-capsular extractive operation and in intra-ocular lens implantation, a suitable pupil dilatation and the maintainment thereof is requested. Because of it, a mydriatic agent , such as a sympathomimetic (e.g. phenylephrine, adrenaline, ephedrine, amphetamine, hydroxyamphetamine) or an anti-muscarine (e.g. atropine, scopolamine, homatropine, cyclopentolate, tropicamide, eucatropine) compound is administered at a certain moment before the operation, for example, about 1.5 hours before in the case of atropine.

It has been now surprisingly found that naproxen sodium salt is effective in maintaining the eye mydriasis set up by the above compounds, specifically during operation.

Sodium naproxen eye drops can be instilled before, during and/or after the mydriatic agent, but this must take place at least 30 minutes before the operation.

The present invention relates to the use of naproxen sodium salt for the manufacture of a medicine useful in the maintainment of eye mydriasis.

The amount of naproxen sodium salt for the use of the invention is from 0.1% to 2% w/v (wherein the weight is intended in gram over a volume intended in liter), preferably of 0.5% w/v. The composition for the use of the invention optionally also comprises at least an excipient selected from the group consisting of at least a viscosity-inducing agent, an isotonicity-causing agent making the ophthalmic solution to be isotonic with the lacrimal liquid, a preservative, a stabilizing agent and a buffer useful for keeping the solution pH as near as possible to the physiologic one.

As viscosity-inducing agents, cellulose derivatives, polyvinylalcohol, polyvinylpyrrolidone, hyaluronic acid, etc. may be listed.

As isotonicity-causing agents, sodium chloride, potassium chloride, mannitol, etc. may be mentioned.

As stabilizing agents, sodium metabisulphite, L-ascorbic acid, sodium ethylenediaminotetraacetate, etc. may be mentioned.

As buffers, citrate, phosphate, borate buffer, etc. may be mentioned.

As preservatives being particularly advantageously used when the ophthalmic composition is prepared in multi-dose container, benzalkonium chloride, thimerosal, chlorhexidine gluconate, phenylethyl alcohol, etc. may be mentioned.

The activity of the ophthalmic composition for the use of the present invention containing naproxen sodium salt will be now shown in further detail by data from tests carried out by the inventors.

EXAMPLE

Four New Zealand albine male rabbits having an average weight of 3.01 kg were housed at optimal temperature, moisture and light conditions. Before starting the test, the animals were checked for not showing conjunctiva or other ocular tissue inflammation.

A pre-treatment was effected with a 0.5% w/v naproxene sodium salt ophthalmic solution (100 $\mu$l) administered in the rabbit right eye 180, 120, 90 and 30 minutes before carrying out paracentesis. The treatment was repeated just after the paracentesis and 15, 75 and 90 minutes thereafter. The left eye was considered as control.

One hour and half before carrying out paracentesis, both the rabbit eyes were instilled with 1% atropine sulfate (40 $\mu$l per eye).

Before the paracentesis operation, the animals were intravenously anesthetized with 20 mg/ml of ketamine hydrochloride and instilled with 2 drops of 0.4% w/v oxybuprocaine hydrochloride collyrium.

The sampling of aqueous humor was effected by means of a 25 G needle connected to a 15 cm lenght of 0,38 mm bore polythene tubing and filled with an eparine physiological solution (50 U.I./ml), the needle

3

being in its turn connected with a syringe. The needle was introduced into the anterior chamber through the cornea taking care not to damage the iris, and about 250 $\mu$l of aqueous humor were removed per each eye.

The pupil diameter measurement was determined with fine dividers and by means of a magnifier, before and 105 minutes after paracentesis, always in the same light conditions.

The experimental data are shown in Tables 1 and 2.

A comparison between the control eyes group and the treated eyes group data was effected by employing t-test Student (biostatistic method). A substantial difference for $p < 0.05$ and $p < 0.001$ ($p$ = probability) was considered. Further the percentage of rabbit eye mydriasis was valued immediately before and 105 minutes after the parecentesis operation, by means of the following formula:

$$\text{mydriasis \%} = \frac{\begin{vmatrix} \text{pupillary diameter} \\ \text{at each time} \\ \text{after atropine} \\ \text{instillation} \end{vmatrix} - \begin{vmatrix} \text{pupillary diameter} \\ \text{before atropine} \\ \text{instillation} \end{vmatrix}}{|\text{pupillary diameter before atropine instillation}|} \times 100$$

## TABLE 1

Pupil diameter in mm (average+Standard Error) of the treated eye (in the beginning with 0.5% naproxene sodium salt collyrium only; 90 minutes before paracentesis with 0.5% naproxene sodium salt collyrium and 1% atropine sulfate collyrium; after paracentesis with 0.5% naproxene sodium salt collyrium only) and of the controls (90 minutes before paracentesis with 1% atropine sulfate collyrium only; no treatment after paracentesis).

| GROUPS | PUPIL DIAMETER (mm) | | |
|---|---|---|---|
| | 180' before paracentesis | immediately before paracentesis | 105' after paracentesis |
| TREATED | 6.00±0.40 | 8.75±0.40 | 8.50±0,15* |
| CONTROL | 6.25±0.40 | 8.50±0.29 | 6.50±0.29 |

* Significantly different from control : $p < 0.05$

4

## TABLE 2

Mydriasis percentage of rabbit eye immediately before and 105 minutes after the paracentesis operation.

| GROUPS | MYDRIASIS % | |
|---|---|---|
| | Immediately before paracentesis | 105' after para-centesis |
| TREATED | 45.83% | 41.6% * |
| CONTROL | 36.00% | 4.00% |

* Significantly different from control; $p < 0.01$

The data taken during the study and shown in Tables 1 and 2 point out the following. The pupil diameter of the control eyes and of the treated eyes, which is of $6.25 \pm 0.40$ mm and $6.00 \pm 0.40$ mm, respectively, at the beginning of the test, increases 90 minutes after 1% atropine sulfate instillation, and is of $8.50 \pm 0.29$ mm and $8.75 \pm 0.40$ mm respectively immediately before paracentesis.

After paracentesis the pupil diameter of controls decreases ($6.50 \pm 0.29$ mm), whereas in the treated eyes it is of $8.50 \pm 0.15$ mm. The latter value is higher than the one of the control eyes and with a significant difference ($p < 0.05$).

The percentages of the mydriasis rate shown in Table 2 much better exhibit differences between the treated and control groups and confirm the significant difference ($p < 0.001$) after paracentesis.

The results of the present experiments confirm the myosis inhibition subsequent to the surgical trauma, and consequently the mydriasis maintainment in the eyes treated with naproxene sodium salt. Unlike the controls, wherein the pupil diameter return to almost normal values after 105 minutes after paracentesis, in the treated eyes a mydriasis higher than 40% may be detected.

## Claims

**Claims for the following Contracting States : AT, BE, CH, DE, DK, FR, GB, IT, LI, LU, NL, SE**

1. Use of naproxen sodium salt for the manufacture of a medicine useful in maintaining the eye mydriasis.

2. Use of naproxen sodium salt according to claim 1 characterised in that said medicine comprises an effective amount of naproxene sodium salt in association with an ophthalmically acceptable oily or aqueous carrier.

3. Use of naproxen sodium salt according to the previous claims characterised in that said medicine comprises an amount of the naproxene sodium salt from 0.1 to 2.0% w/v.

4. Use of naproxene sodium salt according to the previous claims characterised in that said medicine comprises at least a viscosity-inducing agent, an isotonicity-causing agent, a preservative, a stabilizing agent and a buffer.

EP 0 456 988 B1

**Claims for the following Contracting States : GR, ES**

1.  Process for the preparation of an ophthalmic composition for use of naproxen sodium salt in the maintainment of eye mydriasis, characterized in that naproxen sodium salt as active ingredient is dissolved in an ophthalmically acceptable oily or acqueous carrier in an amount of from 0.1% to 2% w/v.

2.  A process according to claim 1, characterized in that the active ingredient is dissolved in an amount of about 0.5% w/v.

3.  A process according to claims 1 and 2 characterized in that at least an excipient selected from the group consisting of at least a viscosisty-inducing agent, an isotonicity-causing agent that makes the solution to be isotonic with the lacrimal liquid, a preservative, a stabilizing agent and a buffer for adjusting the pH of the solution to the physiologic one, is added to the active ingredient.

**Patentansprüche**
**Patentansprüche für folgende Vertragsstaaten : AT, BE, CH, DE, DK, FR, GB, IT, LI, LU, NL, SE**

1.  Verwendung des Naproxennatriumsalzes für die Herstellung eines Arzneimittels, das nützlich ist, um die Augenmydriasis aufrechtzuerhalten.

2.  Verwendung des Naproxennatriumsalzes nach Anspruch 1, dadurch **gekennzeichnet,** daß das Arzneimittel eine wirksame Menge an Naproxennatriumsalz zusammen mit einem augenannehmbaren öligen oder wäßrigen Träger enthält.

3.  Verwendung des Naproxennatriumsalzes entsprechend den vorhergehenden Ansprüchen, dadurch **gekennzeichnet,** daß das Arzneimittel eine Menge an Naproxennatriumsalz von 0,1 bis 2,0 % Gew./Vol. enthält.

4.  Verwendung des Naproxennatriumsalzes entsprechend den vorhergehenden Ansprüchen, dadurch **gekennzeichnet,** daß das Arzneimittel mindestens ein viskositätsinduzierendes Mittel, ein Mittel zur Einstellung der Isotonizität, ein Konservierungsmittel, einen Stabilisator und einen Puffer enthält.

**Patentansprüche für folgende Vertragsstaaten : GR, ES**

1.  Verfahren zur Herstellung einer Augenzusammensetzung für die Verwendung des Naproxennatriumsalzes bei der Aufrechterhaltung der Augenmydriasis, dadurch **gekennzeichnet,** daß das Naproxennatriumsalz als aktiver Bestandteil in einem augenannehmbaren öligen oder wäßrigen Träger in einer Menge von 0,1 bis 2,0 % Gew./Vol. gelöst wird.

2.  Verfahren nach Anspruch 1, dadurch **gekennzeichnet,** daß der aktive Bestandteil in einer Menge von etwa 0,5 % Gew./Vol. gelöst wird.

3.  Verfahren nach einem der Ansprüche 1 und 2, dadurch **gekennzeichnet,** daß mindestens ein Excipient, ausgewählt aus der Gruppe, bestehend aus mindestens einem viskositätsinduzierenden Mittel, einem Mittel, das Isotonizität bewirkt und das bewirkt, daß die Lösung mit der Tränenflüssigkeit isotonisch wird, einem Konservierungsmittel, einem Stabilisator und einem Puffer für die Einstellung des pHs der Lösung auf einen physiologischen Wert, zu dem aktiven Bestandteil zugegeben wird.

**Revendications**
**Revendications pour les Etats contractants suivants : AT, BE, CH, DE, DK, FR, GB, IT, LI, LU, NL, SE**

1.  Utilisation du sel de sodium du naproxène pour préparer un médicament utile au maintien de la mydriase de l'oeil.

2.  Utilisation du sel de sodium du naproxène selon la revendication 1, caractérisée en ce que ce médicament comprend une quantité efficace du sel de sodium du naproxène, en association avec un excipient huileux ou aqueux acceptable d'un point de vue ophtalmique.

6

**3.** Utilisation du sel de sodium du naproxène selon les revendications précédentes, caractérisée en ce que le médicament comprend une quantité du sel de sodium du naproxène de 0,1 à 2,0 % m/V.

**4.** Utilisation du sel de sodium du naproxène selon les revendications précédentes, caractérisée en ce que le médicament comprend au moins un agent de viscosité, un agent d'isotonicité, un conservateur, un stabilisant et un tampon.

**Revendications pour les Etats contractants suivants : GR, ES**

**1.** Procédé pour préparer une composition ophtalmique pour utiliser le sel de sodium du naproxène dans le maintien de la mydriase de l'oeil, caractérisé en ce qu'on dissout le sel de sodium du naproxène, servant de principe actif, dans un excipient huileux ou aqueux acceptable d'un point de vue ophtalmique, en une quantité de 0,1 à 2 % m/V.

**2.** Procédé selon la revendication 1, caractérisé en ce que le principe actif est dissout en une quantité d'environ 0,5 % m/V.

**3.** Procédé selon les revendications 1 et 2, caractérisé en ce qu'on ajoute au principe actif au moins un excipient choisi parmi l'ensemble comprenant au moins un agent de viscosité, un agent d'isotonicité qui rend la solution isotonique du liquide lacrymal, un conservateur, un stabilisant et un tampon pour ajuster le pH de la solution au pH physiologique.